# EUROPÄISCHE PATENTSCHRIFT

(11) **EP 1 071 653 B1**
(45) Veröffentlichungstag und Bekanntmachung des Hinweises auf die Patenterteilung: **20.08.2003**
(21) Anmeldenummer: 99908940.2
(22) Anmeldetag: 23.02.1999
(51) Int. Cl.: C07C 209/48, B01J 23/78, B01J 23/889

(54) **KATALYSATOR AUF DER BASIS VON EISEN ZUR HYDRIERUNG VON ALPHA, OMEGA-DINITRILEN**
IRON-BASED CATALYST FOR HYDROGENATING ALPHA-, OMEGA-DINITRILES
CATALYSEUR A BASE DE FER POUR L'HYDROGENATION DE DINITRILES ALPHA,OMEGA

(30) Priorität: 06.03.1998 DE 19809687
(43) Veröffentlichungstag der Anmeldung: 31.01.2001
(73) Patentinhaber: BASF AKTIENGESELLSCHAFT, 67056 Ludwigshafen (DE)
(72) Erfinder: VOIT, Guido, D-67251 Freinsheim (DE); FISCHER, Rolf, D-69121 Heidelberg (DE); BASSLER, Peter, D-68519 Viernheim (DE); ANSMANN, Andreas, D-69168 Wiesloch (DE); LUYKEN, Hermann, D-67069 Ludwigshafen (DE); MERGER, Martin, D-67227 Frankenthal (DE); OHLBACH, Frank, D-69221 Dossenheim (DE); REHFINGER, Alwin, D-67112 Mutterstadt (DE)
(86) Internationale Anmeldenummer: EP9901150
(87) Internationale Veröffentlichungsnummer: WO99044984

(56) Entgegenhaltungen:
- WO-A-98/11059
- DE-A- 2 429 293

## Beschreibung

Die vorliegende Erfindung betrifft ein Verfahren zur Hydrierung von alpha,omega-Dinitrilen in Gegenwart eines Katalysators, dadurch gekennzeichnet, daß man als Katalysator eine Masse, enthaltend
(a) Eisen oder eine Verbindung auf der Basis von Eisen oder deren Gemische,
(b) von 0,001 bis 0,3 Gew. % bezogen auf (a) eines Promotors auf der Basis von 2, 3, 4 oder 5 Elementen ausgewählt aus der Gruppe bestehend aus Aluminium, Silizium, Zirkonium, Titan, Vanadium,
(c) von 0 bis 0,3 Gew. % bezogen auf (a) einer Verbindung auf der Basis eines Alkali- und/oder Erdalkalimetalls, sowie
(d) von 0,001 bis 0,3 Gew. % bezogen auf (a) Mangan
einsetzt.

Ferner betrifft die Erfindung die Verwendung solcher Massen als Katalysator bei der Hydrierung von aliphatischen alpha-, omega-Dinitrilen und solche Massen, erhältlich nach bestimmten Verfahren.

Es ist allgemein bekannt, beispielsweise aus Weissermel/Arpe, Industrielle Organische Chemie, Verlag Chemie, dritte Auflage, 1988, Seite 266, und WO-A-96/20166 Adipodinitril im Gegenwart von Ammoniak unter Hochdruckbedingungen an überwiegend Eisen enthaltenden Katalysatoren zu 6-Aminocapronitril und/oder Hexamethylendiamin, die beide wichtige Vorprodukte für die Herstellung von Polyamiden wie Nylon 6 und Nylon 6.6 darstellen, zu hydrieren.

Wichtig für optimale Eisenkatalysatoren ist eine hohe mechanische Festigkeit, eine lange Katalysatorstandzeit, eine hohe Raum-Zeit-Ausbeute an den Wertprodukten alpha, omega-Aminonitril und/oder alpha, omega-Diamin bei vollständigem alpha, omega-Dinitril-Umsatz bei möglichst geringem Gehalt an unerwünschten Nebenprodukten.

Diese unerwünschten Nebenprodukte entstehen je nach Katalysator in unterschiedlichen Mengen und lassen sich nur mit hohem Aufwand von dem als Wertprodukt entstehenden Aminonitril und/oder Diamin abtrennen.

So bilden sich beispielsweise im Falle der Hydrierung von Adipodinitril zu Hexamethylendiamin in wechselnden Mengen unter anderem Tetrahydroazepin (THA), 1-Amino-2-cyanocyclopenten (ICCP), 2-Aminomethylcyclopentylamin (AMCPA), 1,2-Diaminocyclohexan (DCH) und Bishexamethylentriamin (BHMTA). Aus der US-A 3 696 153 ist bekannt, daß sich AMCPA und DCH nur sehr schwer von Hexamethylendiamin abtrennen lassen. Vor allem große Mengen an AMCPA, DCH und THA führen zu einem hohen Destillationsaufwand, der sich in beträchtlichen Investitions- und Energiekosten niederschlägt.

Aus US-A-4.282.381, Spalte 2, Tabelle 1, ist bekannt, daß bei Hydrierung von Adipodinitril zu Hexamethylendiamin in Gegenwart von Eisenkatalysatoren als Nebenprodukt unter anderem durchschnittlich 2400 bis 4000 ppm 1,2-Diaminocyclohexan, 100 bis 300 ppm 2 Aminomethylcyclopentylamin, 200 bis 900 ppm Tetrahydroazepin und 2000 bis 5000 ppm 6-Aminocapronitril entstehen.

Aus DE-A-2 429 293, Beispiel 1, ist bekannt, daß bei der Hydrierung von Adipodinitril in Gegenwart der fünffachen Gewichtsmenge Ammoniak bei 93 bis 98°C (Eintrittstemperatur in den Reaktor) bzw. 94 bis 104°C (Austrittstemperatur) an einem mit Aluminiumoxid, Siliziumdioxid, Calciumoxid und Vanadinpentoxid dotierten Eisenkatalysator, der aus Magentit durch Reduktion mit Wasserstoff hergestellt wurde, 98,22 % Hexamethylendiamin mit einem Gehalt von 1900 ppm 1.2 Diaminocyclohexan erhalten werden. Aus Beispiel 2 ist bekannt, daß bei der Hydrierung von Adipodinitril in Gegenwart der fünffachen Gewichtsmenge Ammoniak bei 93 bis 98°C (Eintrittstemperatur in den Reaktor) bzw. 94 bis 104°C (Austrittstemperatur) an einem mit Aluminiumoxid, Siliziumdioxid und Calciumoxid dotierten Eisenkatalysator, der aus einem Labrador-Hämatiterz (Fe₂O₃) durch Reduktion mit Wasserstoff hergestellt wurde, 98,05 % Hexamethylendiamin mit einem Gehalt von 3500 ppm 1.2 Diaminocyclohexan erhalten werden.

Aufgabe der vorliegenden Erfindung war es daher, Verfahren zur Hydrierung von alpha, omega-Dinitrilen (I) zu alpha, omega-Aminonitrilen (II) und/oder alpha, omega-Diaminen (III) in Gegenwart eines Katalysators sowie Katalysatoren bereitzustellen, die die genannten Nachteile nicht aufweisen und die die Hydrierung von von alpha, omega-Dinitrilen mit hoher Selektivität auf technisch einfache und wirtschaftliche Weise bei hoher Standzeit des Katalysators ermöglichen.

Demgemäß wurden das eingangs definierte Verfahren, die eingangs definierte Verwendung, sowie die eingangs definierten Massen gefunden.

Das erfindungsgemäße Verfahren führt man in Gegenwart von als Katalysator geeignete Massen durch, die vorzugsweise eine BET-Oberfläche von 3 bis 20 m²/g, ein Gesamtporenvolumen von 0,05 bis 0,2 ml/g, einen mittleren Porendurchmesser von 0,03 bis 0,1 µm und einen Porenvolumenanteil im Bereich von 0,01 bis 0,1 µm von 50 bis 70 % aufweisen.

Die Gew. %-Angaben in (b) und (d) beziehen sich auf die Elemente, die Gew. %-Angaben in (c) auf die Oxide der Alkali- und Erdalkalimetalle. Diese Angaben beziehen sich auf die Komponente (a).

Bevorzugte Katalysatorvorläufer sind solche, in denen die Komponente (a) zu 90 bis 100 Gew. %, bevorzugt 92 bis 99 Gew. %, bezogen auf (a) Eisenoxide, Eisenhydroxide, Eisenoxyhydroxide oder deren Gemische enthält. Bevorzugt verwendet werden synthetisch hergestellte oder natürlich vorkommende Eisenoxide, Eisenhydroxide oder Eisenoxyhydroxide, wie Brauneisenstein, Roteisenstein (Hämatit), vorzugsweise Magneteisenstein (Magnetit), der im Idealfall mit der Formel Fe₃O₄ beschrieben werden kann. Das Atomverhältnis von Sauerstoff zu Eisen liegt bevorzugt zwischen 1,25 : 1 bis 1,45 : 1, bevorzugt 1,3 : 1 bis 1.4 : 1, besonders bevorzugt bei 1,33 : 1, also reinem Magnetit.

Wird Magnetit synthetisch hergestellt, so kann man von sehr reinem metallischem Eisen oder von sehr reinen Eisen (II)- und/oder Eisen (III)-Verbindungen ausgehen, denen nachträglich die Dotierungselemente in Form geeigneter Verbindungen zugesetzt werden.

Bevorzugte Katalysatorvorläufer sind weiterhin solche, in denen Komponente (b) von 0,001 bis 0,3 Gew. %, vorzugsweise 0,01 bis 0,2 Gew. %, insbesondere 0,01 bis 0,1 Gew. % eines Promotors auf der Basis von 2, 3, 4 oder 5, vorzugsweise 3, 4 oder 5 Elementen ausgewählt aus der Gruppe bestehend aus Aluminium, Zirkonium, Silizium, Titan oder Vanadium enthält, insbesondere die Kombination Aluminium, Silizium und Titan.

Bevorzugte Katalysatorvorläufer sind weiterhin solche, in denen Komponente (c) von 0 bis 0,3 Gew. %, vorzugsweise 0,01 bis 0,2 Gew. %, besonders bevorzugt 0,01 bis 0,1 Gew. % einer Verbindung auf der Basis eines Alkali-oder Erdalkalimetalls, ausgewählt aus der Gruppe bestehend aus Lithium, Natrium, Kalium, Rubidium, Cäsium, Magnesium und Calcium, vorzugsweise Calcium und/oder Magnesium, enthält.

Die erfindungsgemäß eingesetzten Massen enthalten 0,001 bis 1 Gew. %, vorzugsweise 0,001 bis 0,3 Gew. %, insbesondere 0,01 bis 0,2 Gew. % Mangan

Bei den erfindungsgemäß eingesetzten Katalysatoren kann es sich um Voll- oder Trägerkatalysatoren handeln. Als Trägermaterialien kommen beispielsweise poröse Oxide wie Aluminiumoxid, Siliziumdioxid, Alumosilikate, Lanthanoxid, Titandioxid, Zirkondioxid. Magnesiumoxid, Zinkoxid, und Zeolithe sowie Aktivkohle oder Mischungen davon in Betracht.

Die Herstellung erfolgt in der Regel derart, daß man Vorläufer der Komponente (a) gewünschtenfalls zusammen mit Vorläufern der Promotoren Komponenten (b), (d) und gewünschtenfalls mit Vorläufern der Komponenten (c) in Gegenwart oder Abwesenheit von Trägermaterialien (je nachdem welcher Katalysatortyp gewünscht ist) ausfällt, gewünschtenfalls den so erhaltenen Katalysatorvorläufer zu Strängen oder Tabletten verarbeitet, trocknet und anschließend calciniert. Trägerkatalysatoren sind im allgemeinen auch erhältlich, indem man den Träger mit einer Lösung der Komponenten (a), (b), (d) und gewünschtenfalls (c) tränkt, wobei man die einzelnen Komponenten gleichzeitig oder nacheinander zugeben kann, oder indem man die Komponenten (a), (b), (d) und gewünschtenfalls (c) auf den Träger nach an sich bekannten Methoden aufsprüht.

Als Vorläufer der Komponenten (a) kommen in der Regel gut wasserlösliche Salze des Eisens wie Nitrate, Chloride, Acetate, Formiate und Sulfate, vorzugsweise Nitrate, in Betracht.

Als Vorläufer der Komponenten (b) und (d) kommen in der Regel gut wasserlösliche Salze oder Komplexsalze der zuvor genannten Metalle und Halbmetalle wie Nitrate, Chloride, Acetate, Formiate und Sulfate in Betracht, vorzugsweise Nitrate.

Als Vorläufer der Komponenten (c) kommen in der Regel gut wasserlösliche Salze der zuvor genannten Alkalimetalle und Erdalkalimetalle wie Hydroxide, Carbonate, Nitrate, Chloride, Acetate, Formiate und Sulfate in Betracht, vorzugsweise Hydroxide und Carbonate.

Die Fällung erfolgt im allgemeinen aus wäßrigen Lösungen, wahlweise durch Zugabe von Fällungsreagenzien, durch Änderung des pH-Wertes oder durch Änderung der Temperatur.

Üblicherweise trocknet man die so erhaltene Katalysatorvormasse im allgemeinen bei Temperaturen im Bereich von 80 bis 150°C, vorzugsweise von 80 bis 120°C vor.

Das Calcinieren nimmt man üblicherweise bei Temperaturen im Bereich von 150 bis 500°C, vorzugsweise von 200 bis 450°C in einem Gasstrom aus Luft oder Stickstoff vor.

Nach dem Calcinieren setzt man die erhaltene Katalysatormasse im allgemeinen einer reduzierenden Atmosphäre aus ("Aktivierung"), beispielsweise indem man sie bei einer Temperatur im Bereich von 200 bis 500°C, vorzugsweise von 250 bis 400°C 2 bis 24 Stunden einer Wasserstoffatmosphäre oder einer Gasmischung, enthaltend Wasserstoff und ein Inertgas wie Stickstoff, aussetzt. Die Katalysatorbelastung beträgt hierbei bevorzugt 200 1 pro Liter Katalysator.

Nach DE 24 29 293, Seite 7, Zeilen 1 bis 12, kann es vorteilhaft sein, dem zur Aktivierung verwendeten Wasserstoff Ammoniak zuzusetzen.

Vorteilhaft führt man die Aktivierung des Katalysators direkt im Synthesereaktor durch, da hierdurch üblicherweise ein ansonsten erforderlicher Zwischenschritt, nämlich die Passivierung der Oberfläche bei üblicherweise Temperaturen im Bereich von 20 bis 80°C, vorzugsweise von 25 bis 35°C, mittels Sauerstoff-Stickstoff-Mischungen wie Luft, wegfällt. Die Aktivierung passivierter Katalysatoren nimmt man dann bevorzugt im Synthese-Reaktor bei einer Temperatur im Bereich von 180 bis 500°C, vorzugsweise von 200 bis 350°C, in einer wasserstoffhaltigen Atmosphäre vor.

Die Katalysatoren können als Festbettkatalysatoren in Sumpf- oder Rieselfahrweise oder als Suspensionskatalysatoren eingesetzt werden.

Als Ausgangsstoffe im erfindungsgemäßen Verfahren werden aliphatische alpha, omega-Dinitrile der allgemeinen Formel I,

NC-(CH₂)ₙ-CN I

in der n eine ganze Zahl von 1 bis 10, insbesondere 2, 3, 4, 5 und 6, bedeutet, eingesetzt. Besonders bevorzugte Verbindungen I sind Bernsteinsäuredinitril, Glutarsäuredinitril, Adipinsäuredinitril ("Adipodinitril"), Pimelinsäuredinitril und Korksäuredinitril ("Suberonitril"), ganz besonders bevorzugt Adipodinitril.

Besonders bevorzugt verwendet werden alpha, omega-Dinitrile, die durch Hydrocyanierung in Gegenwart von phosphorhaltigen Katalysatoren eines um zwei Kohlenstoffatome ärmeren alpha, omega-Dienes erhalten wurden, wie Adipodinitril durch Anlagerung von Blausäure an Butadien bzw. 3-Pentennitril in Gegenwart von Nickel (O)-Verbindungen und Triarylphosphiten.

Derartige alpha, omega-Dinitrile können Spuren von phosphorhaltigen Verbindungen, etwa 1 bis 50 ppm, gerechnet als Phoshor und bezogen auf alpha, omega-Dinitril, enthalten. Entfernt man diese Phosphorverbindungen ganz oder teilweise, wobei man Gewichtsanteile an phosphorhaltiger Verbindung von kleiner 5 ppm, vorzugsweise kleiner 1 ppm erhält, so lassen sich die bei dem erfindungsgemäßen Verfahren und der erfindungsgemäßen Verwendung erzielten hohen Katalysatorstandzeiten nochmals steigern.

Zur Reduzierung des Gewichtsanteils an einer phosphorhaltigen Verbindung in dem Gemisch kommen verschiedene, an sich bekannte Verfahren, wie Fällung, vorzugsweise Extraktion, Behandlung mit einer Base wie Natronlauge oder Kalilauge, Adsorption oder Chemisorption, insbesondere an ein Metall wie Eisen oder, besonders bevorzugt, Destillation in Betracht. Besonders bevorzugt ist auch die Behandlung des Dinitrils mit Metallbasen der Alkali- und Erdalkaligruppe, der Lanthaniden und der Gruppen III a, II b und III b des Periodensystems, wie z. B. Calciumoxid.

Die Destillation kann man dabei vorteilhaft bei Drücken von 1 bis 100 mbar, vorzugsweise 10 bis 200 mbar durchführen, wobei das Adipodinitril meist als Kopfprodukt anfällt, da die phosphorhaltigen Verbindungen im wesentlichen schwerflüchtiger als Adipodinitril ist.

Nach dem erfindungsgemäßen Verfahren können die vorstehend beschriebenen Dinitrile I vorzugsweise in Gegenwart eines Lösungsmittels unter Verwendung eines Katalysators zu alpha-, omega-Aminonitrilen der allgemeinen Formel II

NC-(CH₂)ₙ-CH₂-NH₂ II

hydriert werden, wobei n die vorstehend genannte Bedeutung hat. Besonders bevorzugte Aminonitrile II sind solche, in denen n einen Wert von 2, 3, 4, 5 oder 6 hat, insbesondere 4, d. h. 4 Aminobutansäurenitril, 1,5 Aminopentansäurenitril, 1,6 Aminohexansäurenitril ("6-Aminocapronitril"), 1,7-Aminoheptansäurenitril und 1,8 Aminooctansäurenitril, ganz besonders bevorzugt 6 Aminocapronitril.

Führt man die Umsetzung in einer Suspension durch, wählt man üblicherweise Temperaturen im Bereich von 40 bis 150°C, vorzugsweise von 50 bis 100°C, besonders vorzugsweise von 60 bis 90°C. Den Druck wählt man im allgemeinen im Bereich von 2 bis 30 Mpa, vorzugsweise von 3 bis 30 Mpa, besonders bevorzugt von 4 bis 9 Mpa. Die Verweilzeiten sind im wesentlichen von der gewünschten Ausbeute, Selektivität und bei vollständigem Umsatz abhängig; üblicherweise wählt man die Verweilzeit so, daß bei vollständigem Umsatz ein Maximum an Ausbeute erreicht wird, beispielsweise im Bereich von 50 bis 275 min, vorzugsweise von 70 bis 200 min.

Bei der Suspensionsfahrweise setzt man als Lösungsmittel bevorzugt Ammoniak, Amine, Diamine und Triamine mit 1 bis 6 C-Atomen wie Trimethylamin, Triethylamin, Tripropylamin und Tributylamin oder Alkohole, insbesondere Methanol und Ethanol, besonders bevorzugt Ammoniak ein. Zweckmäßig wählt man eine Dinitrilkonzentration im Bereich von 10 bis 90 Gew. %, vorzugsweise von 30 bis 80 Gew. %, besonders vorzugsweise von 40 bis 70 Gew. %, bezogen auf die Summe von Dinitril und Lösungsmittel.

Die Menge an Katalysator wählt man im allgemeinen so, daß die Katalysator-Menge im Bereich von 1 bis 50 Gew.-%, bevorzugt von 5 bis 20 Gew. %, bezogen auf die eingesetzte Menge an Dinitril, beträgt.

Die suspensionshydrierung kann man diskontinuierlich oder, bevorzugt kontinuierlich, in der Regel in der Flüssigphase durchführen.

Man kann die Hydrierung auch diskontinuierlich oder kontinuierlich in einem Festbettreaktor in Riesel- oder Sumpffahrweise in geradem Durchgang oder mit Produktrückführung durchführen, wobei man üblicherweise eine Temperatur im Bereich von 20 bis 150°C, vorzugsweise von 30 bis 90°C und einen Druck in der Regel im Bereich von 2 bis 40 Mpa, vorzugsweise von 3 bis 30 Mpa, wählt. Bevorzugt führt man die Hydrierung in Gegenwart eines Lösungsmittels, bevorzugt Ammoniak, Amine, Diamine und Triamine, mit 1 bis 6 C-Atomen, wie Trimethylamin, Triethylamin, Tripropylamin und Tributylamin, oder Alkohol, bevorzugt Methanol und Ethanol, besonders bevorzugt Ammoniak, durch. In einer bevorzugten Ausführungsform wählt man einen Gehalt an Ammoniak im Bereich von 1 bis 10 g, bevorzugt von 2 bis 6 g pro Gramm Adipodinitril. Bevorzugt wählt man dabei eine Katalysatorbelastung im Bereich von 0,1 bis 2,0 kg, vorzugsweise von 0,3 bis 1,0 kg Adipodinitril/1 x h. Auch hier kann man durch Veränderung der Verweilzeit den Umsatz gezielt einstellen.

Die Hydrierung kann man in einem üblichen hierfür geeigneten Reaktor durchführen.
Das Verhältnis von Aminonitrilen (II) zu Diaminen (III) läßt sich durch gezielte Wahl von Temperatur und Katalysatorbelastung steuern.

Bei der Hydrierung von Adipodinitril als alpha, omega-Dinitril erhält man eine Mischung, die neben dem Lösungsmittel ganz überwiegend 6-Aminocapronitril, Hexamethylendiamin und nicht umgesetztes Adipodinitril enthält, das als Verunreinigungen vor allem Hexamethylenimin, 2-Aminomethylcyclopentylamin, 1.2-Diaminocyclohexan. Tetrahydroazepin und Bishexanmethylentriamin enthalten kann.

Die Abtrennung von 6-Aminocapronitril, Hexamethylendiamin und einem im wesentlichen Adipodinitril enthaltenden Teil von der Mischung kann in an sich bekannter Weise, vorzugsweise destillativ, beispielsweise gemäß DE-A-19 500 222 oder der Deutschen Anmeldung 19 548 289.1, gleichzeitig oder nacheinander erfolgen.

Nach dem erfindungsgemäßen Verfahren können die vorstehend beschriebenen Dinitrile I vorzugsweise in Gegenwart eines Lösungsmittels unter Verwendung eines Katalysators zu alpha-, omega-Diaminen der allgemeinen Formel III

H₂N-CH₂-(CH₂)ₙ-CH₂-NH₂ III

hydriert werden, wobei n die vorstehend genannte Bedeutung hat. Besonders bevorzugte Diamine III sind solche, in denen n einen Wert von 2, 3, 4, 5 oder 6 hat, insbesondere 4, d. h. 4 Diaminobutan. 1,5 Diaminopentan, 1,6 Diaminohexan, ("Hexamethylendiamin"), 1,7-Diaminoheptan und 1,8 Diaminooctan, ganz besonders bevorzugt 1,6 Diaminohexan.

Führt man die Umsetzung in einer Suspension durch, wählt man üblicherweise Temperaturen im Bereich von 60 bis 200°C, vorzugsweise von 60 bis 180°C, besonders vorzugsweise von 70 bis 130°C. Den Druck wählt man im allgemeinen im Bereich von 2 bis 30 Mpa, vorzugsweise von 3 bis 30 Mpa, besonders bevorzugt von 4 bis 20 Mpa. Die Verweilzeiten sind im wesentlichen von der gewünschten Ausbeute und Selektivität bei vollständigem Umsatz abhängig; üblicherweise wählt man die Verweilzeit so, daß bei vollständigem Umsatz ein Maximum an Ausbeute erreicht wird, beispielsweise im Bereich von 50 bis 300 min, vorzugsweise von 70 bis 200 min.

Bei der Suspensionsfahrweise setzt man als Lösungsmittel bevorzugt Ammoniak, Amine, Diamine und Triamine mit 1 bis 6 C-Atomen wie Trimethylamin, Triethylamin, Tripropylamin und Tributylamin oder Alkohole, insbesondere Methanol und Ethanol, besonders bevorzugt Ammoniak ein. Zweckmäßig wählt man eine Dinitrilkonzentration im Bereich von 10 bis 90 Gew. %, vorzugsweise von 30 bis 80 Gew. %, besonders vorzugsweise von 40 bis 70 Gew. %, bezogen auf die Summe von Dinitril und Lösungsmittel.

Die Menge an Katalysator wählt man im allgemeinen so, daß die Katalysator-Menge im Bereich von 1 bis 50 Gew.-%, bevorzugt von 5 bis 20 Gew. %, bezogen auf die eingesetzte Menge an Dinitril, beträgt.

Die Suspensionshydrierung kann man diskontinuierlich oder, bevorzugt kontinuierlich, in der Regel in der Flüssigphase durchführen.

Man kann die Hydrierung auch diskontinuierlich oder kontinuierlich in einem Festbettreaktor in Riesel- oder Sumpffahrweise in geradem Durchgang oder mit Produktrückführung durchführen, wobei man üblicherweise eine Temperatur im Bereich von 70 bis 200°C, vorzugsweise von 80 bis 150°C und einen Druck in der Regel im Bereich von 2 bis 40 Mpa, vorzugsweise von 3 bis 30 Mpa, wählt. Bevorzugt führt man die Hydrierung in Gegenwart eines Lösungsmittels, bevorzugt Ammoniak, Amine, Diamine und Triamine, mit 1 bis 6 C-Atomen, wie Trimethylamin, Triethylamin, Tripropylamin und Tributylamin, oder Alkohol, bevorzugt Methanol und Ethanol, besonders bevorzugt Ammoniak, durch. In einer bevorzugten Ausführungsform wählt man einen Gehalt an Ammoniak im Bereich von 1 bis 10 g, bevorzugt von 2 bis 6 g pro Gramm Adipodinitril. Bevorzugt wählt man dabei eine Katalysatorbelastung im Bereich von 0,1 bis 2,0 kg, vorzugsweise von 0,3 bis 1,5 kg Adipodinitril/1 x h. Auch hier kann man durch Veränderung der Verweilzeit den Umsatz gezielt einstellen.

Die Hydrierung kann man in einem üblichen hierfür geeigneten Reaktor durchführen.

Bei der Hydrierung von Adipodinitril als alpha, omega-Dinitril erhält man eine Mischung, die neben dem Lösungsmittel ganz überwiegend Hexamethylendiamin enthält, das als Verunreinigungen vor allem 6-Aminocapronitril, Hexamethylenimin, 2-Aminomethylcyclopentylamin, 1.2-Diaminocyclohexan, Tetrahydroazepin und Bishexanmethylentriamin enthalten kann.

Die Reinigung des nach Abtrennung des Lösungsmittels erhaltenen Roh-Hexamethylendiamins erfolgt in allgemeinen bevorzugt destillativ.

Die alpha, omega-Aminonitrile und alpha-, omega-Diamine sind wichtige Ausgangsverbindungen zur Herstellung von Nylon 6.6 und/oder Nylon 6.

In den Beispielen bedeuten:
- ADN: = Adipodinitril
- ACN: = 6-Aminocapronitril
- HMD: = Hexamethylendiamin
- DCH: = 1,2-Diaminocyclohexan
- AMCPA: = 2-Aminomethylcyclopentylamin
- BHMTA: = Bis-Hexamethylentriamin
- ICCP: = 1-Amino-2-cyanocylopenten
- THA: = Tetrahydroazepin
- HMI: = Hexamethylenimin

Die in der Tabelle zusammengestellten Analysenwerte wurden durch quantitative Gaschromatographie erhalten.

### Beispiel 1

### a) Katalysatorherstellung

Der Katalysator wurde hergestellt durch sechsstündiges Tempern eines Magnetiterzes bei 1500°C unter Stickstoff. Das verwendete Magnetiterz hatte folgende Zusammensetzung: 72 Gew. % Fe, 0,06 Gew. % Al, 0,03 Gew. % Ca, 0,04 Gew. % Mg, 0,10 Gew. % Si, 0,01 Gew. % Ti, 0,13 Gew.-% Mn, Rest Sauerstoff.

Der abgekühlte Schmelzblock wurde im Backenbrecher zerkleinert und eine Siebfraktion der Teilchengröße 1,5 bis 3 mm ausgesiebt. Der oxidische Katalysator wurde im H₂/N₂-Strom bei 450°C 72 Stunden lang reduziert. Nach Abkühlen unter Stickstoff auf Raumtemperatur wurde der Fe-Katalysator mit einem N₂/Luft-Strom passiviert (24 Stunden mit 1 % Luft in Stickstoff), wobei darauf geachtet wurde, daß die Temperatur im Katalysatorbett nicht über 45°C anstieg.

### b Hydrierung von ADN zu HMD und/oder ACN

Drei in Reihe geschaltete Rohrreaktoren (Gesamtlänge 4,5 m, d = 6 mm) wurden mit 142 ml (240 g) des nach Beispiel 1 a) hergestellten Katalysators (1,5 bis 3 mm Splitt) befüllt und anschließend drucklos im Wasserstoffstrom (200 1/h) reduziert. Hierzu wurde die Temperatur innerhalb von 24 Stunden von 70°C auf 340°C angehoben und anschließend 72 Stunden bei 340°C gehalten. Nach Absenken der Temperatur wurde dem Reaktor bei 250 bar ein Gemisch aus 74 bzw. 148 ml/h ADN (Katalysatorbelastung 0,5 bzw. 1,0 kg ADN/l Kat. x h), 365 ml/h NH3 und 200 Nl/h H₂ zugeführt. Nach 7000 Stunden Laufzeit wurde keine Abnahme der Katalysatoraktivität beobachtet. Unter den in Tabelle 1 aufgeführten Bedingungen wurden in Abhängigkeit von der Temperatur und der Katalysatorbelastung folgende Ergebnisse erhalten (Tabelle 1):

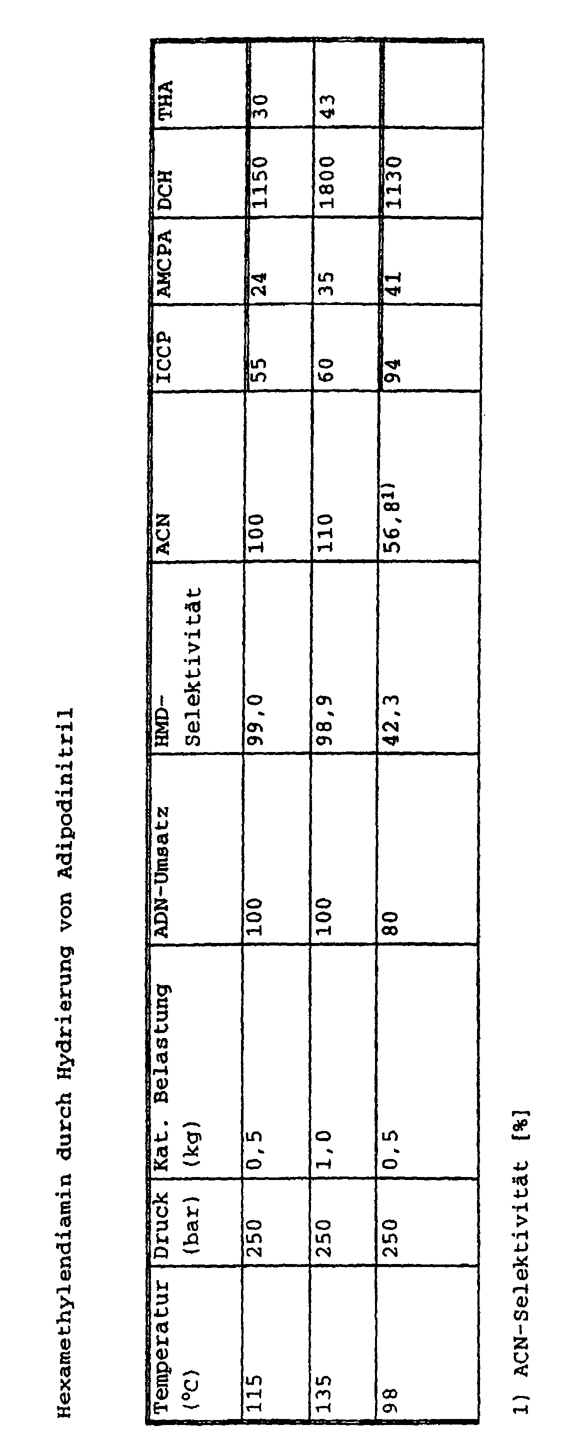

## Patentansprüche

1. Verfahren zur Hydrierung von alpha, omega-Dinitrilen in Gegenwart eines Katalysators, **dadurch gekennzeichnet, daß** man als Katalysator eine Masse, enthaltend
(a) Eisen oder eine Verbindung auf der Basis von Eisen oder deren Gemische,
(b) von 0,001 bis 0,3 Gew. % bezogen auf (a) eines Promotors auf der Basis von 2, 3, 4 oder 5 Elementen ausgewählt aus der Gruppe bestehend aus Aluminium, Silizium, Zirkonium, Titan, Vanadium,
(c) von 0 bis 0,3 Gew. % bezogen auf (a) einer Verbindung auf der Basis eines Alkali- und/oder Erdalkalimetalls, sowie
(d) von 0,001 bis 1 Gew. % bezogen auf (a) Mangan
einsetzt.

2. Verfahren nach Anspruch 1, wobei die Masse eine BET-Oberfläche von 3 bis 20 m²/g, ein Gesamtporenvolumen von 0,05 bis 0,2 ml/g, einen mittleren Porendurchmesser von 0,03 bis 0,1 µm und einen Porenvolumenanteil im Bereich von 0,01 bis 0,1 µm von 50 bis 70 % aufweist.

3. Verfahren nach Anspruch 1 oder 2, wobei die Masse durch Reduktion und gegebenenfalls anschließende Passivierung eines Magnetits erhältlich ist.

4. Verfahren nach den Ansprüchen 1 bis 3, wobei man einen Promoter (b) auf der Basis Aluminium, Silizium und Titan einsetzt.

5. Verfahren nach den Ansprüchen 1 bis 4, wobei man einen Promoter (c) auf der Basis Magnesium und/oder Calcium einsetzt.

6. Verfahren nach den Ansprüchen 1 bis 5, wobei man die Hydrierung in einem Festbettreaktor vornimmt.

7. Verfahren nach den Ansprüchen 1 bis 6, wobei der Katalysator ein Vollkatalysator ist.

8. Verfahren nach den Ansprüchen 1 bis 7, wobei man das alpha, omega-Dinitril zu einem alpha, omega-Diamin hydriert.

9. Verfahren nach Anspruch 8, wobei man als alpha, omega-Dinitril Adipodinitril einsetzt, unter Erhalt von Hexamethylendiamin.

10. Verfahren nach den Ansprüchen 1 bis 7, wobei man das alpha, omega-Dinitril zu einem alpha, omega-Aminonitril hydriert.

11. Verfahren nach Anspruch 10, wobei man als alpha, omega-Dinitril Adipodinitril einsetzt, unter Erhalt von 6-Aminocapronitril.

12. Verfahren nach den Ansprüchen 1 bis 11, wobei eingesetzte alpha- omega-Dinitril durch Hydrocyanierung in Gegenwart von phosphorhaltigen Katalysatoren eines um zwei Kohlenstoffatome ärmeren alpha-, omega-Diens erhalten wurde.

13. Verfahren nach Anspruch 12, **dadurch gekennzeichnet, daß** man den Gewichtsanteil an einer phosphorhaltigen Verbindung, im alpha-, omega-Dinitril reduziert.

14. Verfahren nach Anspruch 12, **dadurch gekennzeichnet, daß** der Gewichtsanteil an einer phosphorhaltigen Verbindung, gerechnet als Phosphor, nach Reduzierung des Gehalts an Phosphorverbindungen, kleiner als 5 ppm, bezogen auf alpha, omega-Dinitril, ist.

15. Verfahren nach Anspruch 12, **dadurch gekennzeichnet, daß** der Gewichtsanteil an einer phosphorhaltigen Verbindung, gerechnet als Phosphor, nach Reduzierung des Gehalts an Phosphorverbindungen, kleiner als 1 ppm, bezogen auf alpha, omega-Dinitril ist.

16. Verwendung von Massen gemäß den Ansprüchen 1 bis 5 als Katalysatoren bei der Hydrierung von alpha-, omega- , Dinitrilen.

17. Masse gemäß den Ansprüchen 1 bis 5, erhältlich durch Reduktion und gegebenenfalls anschließende Passivierung eines Magnetits.

18. Massen gemäß den Ansprüchen 1 bis 5, erhältlich durch Ausfällung von Vorläufern der Komponenten (a), (b), (d) und gewünschtenfalls (c) in Gegenwart oder Abwesenheit von Trägermaterialien.

19. Massen gemäß den Ansprüchen 1 bis 5, erhältlich durch Tränken eines Trägers mit einer Lösung der Komponenten (a), (b), (d) und gewünschtenfalls (c).

20. Massen gemäß den Ansprüchen 1 bis 5, erhältlich durch Aufsprühen der Komponenten (a), (b), (d) und gewünschtenfalls (c) auf einen Träger.

## Claims

1. A process for hydrogenation of alpha, omega-dinitriles in the presence of a catalyst, which comprises using a catalyst comprising a material comprising
(a) iron or a compound based on iron or mixtures thereof,
(b) from 0.001 to 0.3% by weight based on (a) of a promoter based on 2, 3, 4 or 5 elements selected from the group consisting of aluminum, silicon, zirconium, titanium and vanadium,
(c) from 0 to 0.3% by weight based on (a) of a compound based on an alkali and/or alkaline earth metal, and also
(d) from 0.001 to 1% by weight based on (a) of manganese.

2. A process as claimed in claim 1, wherein the material has a BET surface area of from 3 to 20 m²/g, a total pore volume of from 0.05 to 0.2 mL/g, an average pore diameter of from 0.03 to 0.1 µm and a 0.01 to 0.1 µm pore volume fraction within the range from 50 to 70%.

3. A process as claimed in claim 1 or 2, wherein the material is obtainable by reduction with or without subsequent passivation of a magnetite.

4. A process as claimed in any of claims 1 to 3, wherein a promoter (b) based on aluminum, silicon and titanium is used.

5. A process as claimed in any of claims 1 to 4, wherein a promoter (c) based on magnesium and/or calcium is used.

6. A process as claimed in any of claims 1 to 5, wherein the hydrogenation is effected in a fixed bed reactor.

7. A process as claimed in any of claims 1 to 6, wherein the catalyst is an unsupported catalyst.

8. A process as claimed in any of claims 1 to 7, wherein the alpha, omega-dinitrile is hydrogenated to an alpha, omega-diamine.

9. A process as claimed in claim 8, wherein the alpha, omega-dinitrile used is adiponitrile to obtain hexamethylenediamine.

10. A process as claimed in any of claims 1 to 7, wherein the alpha, omega-dinitrile is hydrogenated to an alpha, omega-aminonitrile.

11. A process as claimed in claim 10, wherein the alpha, omega-dinitrile used is adiponitrile to obtain 6-aminocapronitrile.

12. A process as claimed in any of claims 1 to 11, wherein the alpha, omega-dinitrile used was obtained by hydrocyanation in the presence of phosphorus catalysts of an alpha, omega-diene having two carbon atoms fewer.

13. A process as claimed in claim 12, wherein the weight fraction of phosphorus compound in the alpha, omega-dinitrile is reduced.

14. A process as claimed in claim 12, wherein the weight fraction of phosphorus compound, reckoned as phosphorus, is less than 5 ppm, based on alpha, omega-dinitrile, after reduction in the level of phosphorus compounds.

15. A process as claimed in claim 12, wherein the weight fraction of phosphorus compound, reckoned as phosphorus, is less than 1 ppm, based on alpha, omega-dinitrile, after reduction in the level of phosphorus compounds.

16. The use of materials as set forth in any of claims 1 to 5 as catalysts in the hydrogenation of alpha, omega-dinitriles.

17. A material as set forth in any of claims 1 to 5, obtainable by reduction with or without subsequent passivation of a magnetite.

18. A material as set forth in any of claims 1 to 5, obtainable by precipitating precursors of said components (a), (b), (d) and optionally (c) in the presence or absence of support materials.

19. A material as set forth in any of claims 1 to 5, obtainable by impregnating a support with a solution of said components (a), (b), (d) and optionally (c).

20. A material as set forth in any of claims 1 to 5, obtainable by spraying said components (a), (b), (d) and optionally (c) onto a support.

## Revendications

1. Procédé d'hydrogénation de dinitriles alpha,oméga en présence d'un catalyseur, **caractérisé en ce que**, comme catalyseur, on met en oeuvre une masse contenant
(a) du fer ou un composé à base de fer ou leurs mélanges,
(b) de 0,001 à 0,3% en poids, par rapport à (a), d'un activateur à base de 2, 3, 4 ou 5 éléments choisis parmi le groupe constitué de l'aluminium, du silicium, du zirconium, du titane, du vanadium,
(c) de 0 à 0,3% en poids, par rapport à (a), d'un composé à base d'un métal alcalin et/ou alcalino-terreux, ainsi que
(d) de 0,001 à 1% en poids, par rapport (a), de manganèse.

2. Procédé suivant la revendication 1, dans lequel la masse présente une surface BET de 3 à 20 m²/g, un volume poreux global de 0,05 à 0,2 ml/g, un diamètre moyen des pores de 0,03 à 0,1 µm et une fraction volumique des pores dans la gamme de 0,01 à 0,1 µm de 50 à 70%.

3. Procédé suivant l'une des revendications 1 et 2, dans lequel la masse peut être obtenue par réduction et éventuellement passivation ultérieure d'une magnétite.

4. Procédé suivant l'une des revendications 1 à 3, dans lequel on met en oeuvre un activateur (b) à base d'aluminium, de silicium et de titane.

5. Procédé suivant l'une des revendications 1 à 4, dans lequel on met en oeuvre un activateur (c) à base de magnésium et/ou de calcium.

6. Procédé suivant l'une des revendications 1 à 5, dans lequel on effectue l'hydrogénation dans un réacteur à lit fixe.

7. Procédé suivant l'une des revendications 1 à 6, dans lequel le catalyseur est un catalyseur massique.

8. Procédé suivant l'une des revendications 1 à 7, dans lequel on hydrogène le dinitrile alpha,oméga en une diamine alpha,oméga.

9. Procédé suivant la revendication 8, dans lequel, comme dinitrile alpha,oméga, on met en oeuvre de l'adipodinitrile, avec obtention d'hexaméthylènediamine.

10. Procédé suivant l'une des revendications 1 à 7, dans lequel on hydrogène le dinitrile alpha,oméga en un aminonitrile alpha,oméga.

11. Procédé suivant la revendication 10, dans lequel, comme dinitrile alpha,oméga, on met en oeuvre de l'adipodinitrile, avec obtention de 6-aminocapronitrile.

12. Procédé suivant l'une des revendications 1 à 11, dans lequel on obtient le dinitrile alpha,oméga mis en oeuvre par hydrocyanation, en présence de catalyseurs contenant du phosphore, d'un diène alpha,oméga plus pauvre de deux atomes de carbone.

13. Procédé suivant la revendication 12, **caractérisé en ce qu'**on réduit la fraction pondérale en un composé contenant du phosphore dans le dinitrile alpha,oméga.

14. Procédé suivant la revendication 12, **caractérisé en ce que** la fraction pondérale en un composé contenant du phosphore, calculée sous la forme de phosphore, est après réduction de la teneur en composés de phosphore inférieure à 5 ppm, par rapport au dinitrile alpha,oméga.

15. Procédé suivant la revendication 12, **caractérisé en ce que** la fraction pondérale en un composé contenant du phosphore, calculée sous la forme de phosphore, est après réduction de la teneur en composés de phosphore inférieure à 1 ppm, par rapport au dinitrile alpha,oméga.

16. Utilisation de masses suivant l'une des revendications 1 à 5, comme catalyseurs au cours de l'hydrogénation de dinitriles alpha,oméga.

17. Masses suivant l'une des revendications 1 à 5, que l'on peut obtenir par réduction et éventuellement passivation ultérieure d'une magnétite.

18. Masses suivant l'une des revendications 1 à 5, que l'on peut obtenir par précipitation de précurseurs des composants (a), (b), (d) et éventuellement (c), en présence ou en absence de matières de support.

19. Masses suivant l'une des revendications 1 à 5, que l'on peut obtenir par imprégnation d'un support par une solution des composants (a), (b), (d) et éventuellement (c).

20. Masses suivant l'une des revendications 1 à 5, que l'on peut obtenir par pulvérisation des composants (a), (b), (d) et éventuellement (c) sur un support.
